Europäisches Patentamt

European Patent Office

Office européen des brevets

(19)

(11) **EP 0 635 126 B1**

(12) **EUROPEAN PATENT SPECIFICATION**

(45) Date of publication and mention
of the grant of the patent:
**14.07.1999 Bulletin 1999/28**

(21) Application number: **93907994.3**

(22) Date of filing: **05.04.1993**

(51) Int Cl.[6]: **G01N 15/14**

(86) International application number:
**PCT/GB93/00710**

(87) International publication number:
**WO 93/21511 (28.10.1993 Gazette 1993/26)**

(54) **DETECTION OF MICROORGANISMS AND DETERMINATION OF THEIR SENSITIVITY TO ANTIBIOTICS**

NACHWEIS VON MIKROORGANISMEN UND BESTIMMUNG VON DEREN EMPFINDLICHKEIT GEGEN ANTIBIOTIKA

DETECTION DE MICROORGANISMES ET DETERMINATION DE LEUR SENSIBILITE AUX ANTIBIOTIQUES

(84) Designated Contracting States:
AT BE CH DE DK ES FR GB GR IE IT LI LU MC NL PT SE

(30) Priority: **08.04.1992 IL 10155292**

(43) Date of publication of application:
**25.01.1995 Bulletin 1995/04**

(73) Proprietors:
• **Combact Diagnostic Systems Ltd.**
**Harzlia 46766 (IL)**
• **RAMOT UNIVERSITY**
**AUTHORITY FOR APPLIED RESEARCH & INDUSTRIAL DEVELOPMENT LTD.**
**Tel Aviv 69975 (IL)**

(72) Inventors:
• **SCHORR, Amir**
**62 917 Tel Aviv (IL)**
• **SAHAR, Elhanan**
**Tel Aviv (IL)**

(74) Representative: **Marchant, James Ian et al**
**Elkington and Fife,**
**Prospect House,**
**8 Pembroke Road**
**Sevenoaks, Kent TN13 1XR (GB)**

(56) References cited:
• **APPLIED OPTICS vol. 26, no. 16, 15 August 1987, NEW YORK US pages 3280 - 3293 J.W. BACUS 'Optical microscope system for standardized cell measurements and analyses'**
• **PROCEEDINGS OF THE SEVENTH ANNUAL CONFERENCE OF THE IEEE/ENGINEERING IN MEDICINE AND BIOLOGY SOCIETY, CHICAGO, US 27-30 Sept. 1985, vol. 2, pages 980 - 985 B. JAGGI et al.: 'The design and development of an optical scanner for cell biology'**
• **CYTOMETRY vol. 9, no. 3, May 1988, NEW YORK US pages 195 - 200 J.P. PANNO 'Symmetry analysis of cell nuclei'**
• **ZEISS INFORMATION vol. 29, no. 98, July 1987, OBERKOCHEN DE pages 41 - 53 J. KUKULIES ET AL. 'Fluorescent analog cytochemistry of living cells'**
• **IECON'90: 16th ANNUAL CONFERENCE OF IEEE INDUSTRIAL ELECTRONICS SOCIETY, PACIFIC GROVE, US, 27-30 November 1990 vol. I, pages 552 - 557 T. FUKUDA et al: 'Vision system for animal cell recognition in a bio-engineering process'**
• **JOURNAL OF HISTOCHEMISTRY AND CYTOCHEMISTRY vol. 34, no. 1, January 1986, NEW YORK US pages 67 - 74 K. PRESTON 'High resolution image analysis'**

**Description**

## FIELD OF THE INVENTION

The present invention is generally in the field of microbiology and more specifically concerns microbiological assays. In particular, the present invention provides a computerised image analysis method for detecting microorganisms in a biological sample. Furthermore, the present invention provides a computerised image analysis method for assaying the sensitivity of microorganisms to antibiotic drugs.

Also provided by the present invention is a system for carrying out the method of the invention.

## BACKGROUND OF THE INVENTION AND PRIOR ART

The prior art considered to be relevant as a background to the present invention is listed in the end of the description before the claims. These prior art references will be acknowledged by indicating in brackets their number in this list.

Microbiological tests have a wide range of applicability such as in medicine, in monitoring water quality, in assuring food safety etc. In clinical practice microbiological assays are of particular importance and have a major role in the diagnosis of infections and/or determination of a proper drug treatment in case of infection. In such assays the presence of pathological microorganisms in clinical samples, their number, their identity as well as their sensitivity to antibiotic drugs are determined.

The most widely used microbiological assays involve incubating a biological sample on various growth media and determining the growth of microorganisms thereon (1). In order to determine sensitivity of the microorganisms to drug growth of the microorganisms is determined in the presence of the drug (2). Among the biggest drawbacks of such assays is the fact that they require a considerable time, usually 24 to 48 hours, and thus do not enable a rapid diagnosis which is required in cases where immediate treatment is necessary. Thus very often physicians are compelled to begin treatment without waiting for the clinical results which thus have only a confirmatory value. Consequently the treatment is not always appropriate and is also given at times to non-infected patients.

Additionally, such assays are labour consuming and require a considerable amount of laboratory space and this considerably limits the widespread use of such assays in screening of susceptible populations for the occurrence of infections. For example, urinary tract infections are not always accompanied by clinical symptoms (5) and elderly people in which asymptomatic infections may be dangerous (5,6,7), may not be routinely tested.

It may thus be readily understood that methods which do not involve prolonged incubation periods and in which the detection, identification and determination of the sensitivity of microorganisms to antibiotic drugs is thus rapid, would be highly desirable.

There are a number of rapid methods and systems for detecting microorganisms and determining their sensitivity to antibiotic drugs which are currently available (see for example 8,9,10,11,12,13,14,15). By one type of such methods and systems the detection of the microorganisms and the determination of their sensitivity to antibiotic drugs, involves a certain initial incubation period of up to about 24 hours. In addition to the time involved, such methods and systems suffer from serious drawbacks in that they give rise to a large proportion of false positive as well as false negative results. Furthermore, these methods and systems are often not sensitive to concentrations of microorganisms below about $10^4$ or sometimes even $10^5$ microorganisms/ml. Examples of assays which belong to this group are the Autobac System (16,17), the Auto Microbic System (18,19,20), the Impedance System (21) and the Bactec System (22).

Another group of such methods do not require an initial incubation period and therefore are more rapid. However, all these methods have a serious drawback in that they do not enable the determination of sensitivity of the microorganisms to antibiotic drugs and in that, similarly as above, they have a problem of a high rate of false positive and false negative results and they are incapable of detecting microorganisms in concentrations of less than about $10^4$-$10^5$ microorganisms/ml. Examples of such assays are the Bac-t-Screen (19,20,23,24), the Leucocyte Esterase Activity Assay (19,23,25), the Nitrite Test (19,23,26) and the ATP Assay (26,23,27).

It should be noted that all methods described above involve the determination of a single average trait of the bacterial population, a fact giving rise to many of these method's drawbacks. A method which will test several traits on the level of single microorganismal cells has the potential of being much more sensitive in the identification and characterisation of microorganisms in a biological sample.

In addition to the above prior art relating to diagnostic microbial assays, Bacus *et al,* Applied Optics, 26, 3280-3293 (1987) discloses an optical microscope which allows automatic cell measurement and analysis. The system disclosed is intended to differentiate between different cell populations based on the different optical parameters measured by the system and is based on determining the amount, quantity or mass of cell constituents. However, the system cannot and is not intended to discriminate between a microorganism and non-microorganismal objects but rather a sample which is known to consist of cells is analysed to differentiate between different cell populations.

OBJECTS OF THE PRESENT INVENTION

It is the object of the present invention to provide a computerized image analysis method and system by which microorganisms in a biological sample may be rapidly and reliably detected and characterised.

GENERAL DESCRIPTION OF THE INVENTION

The present invention provides a computerised image analysis method by which a specimen is placed in a microscope, the sample's image is recorded, e.g. by a video camera, a grid of photovoltaic cells or on a photographic film, digitised, possibly stored and analysed either on- or off-line. On the basis of the analysis microorganisms are identified, enumerated and characterised.

In analysing a microscopic image for the detection of microorganisms, it is first necessary to distinguish between microorganismal cells and other particulate materials of similar size which may be present in the specimen such as tissue debris, dust particles, protein and sugar aggregates etc. Very often the overall optical properties of such non-microorganismal particles as well as their shape is similar to that of certain microorganisms and therefore in using conventional image analysis techniques, a large percentage of such object may be classified as microorganisms or alternatively, a large number of microorganisms may be characterised as being non-microorganismal objects which will thus mean either an over-count or an under-count of the microorganisms, respectively. This fact is the main cause of the failure hitherto to use image analysis techniques in microbiological assays.

As known, an object viewed under the microscope is not homogeneous in its brightness and various parts of the object may differ from one another in their brightness. This non-homogeneity results from the difference in this object's composition between different parts as well as from the object's shape. It was surprisingly found in accordance with the present invention that microorganisms differ from non-microorganismal objects in the spacial distribution of brightness in an object. A particular example is the rate or gradient of change in brightness across the object's edge (between the interior to the exterior of the object), which was found to differ significantly between microorganisms and non-microorganismal objects of the same size. The degree of change in brightness across an object's edge will be referred to hereinafter as the "**Optical Slope**". Another example is a parameter to be referred to hereinafter as "**moment**" which is a product of the light intensity at each point and of the distance of this point from the object's edge.

The present invention thus provides a computerised image analysis method for detecting microorganisms in a sample comprising placing a specimen from the sample in a microscope, recording the image viewed through the microscope by appropriate image recording means, analysing the image to identify particulate objects therein and classifying these objects as microorganisms or non-microorganismal objects, which classifying comprises determining the optical slope being the change in brightness across the edge of the object.

The spacial distribution of brightness of microorganismal objects in general and the optical slope in particular falls within a certain range depending on various factors such as, the type of illumination and its intensity. For example, in transmitted light illumination, where a very bright or a very dim illuminating light source is being used, the general contrast in the image viewed through the microscope is smaller than where the illuminating light has a medium range and hence the optical slope of objects under bright or dim illumination are smaller than under conditions of medium range intensity illumination. Where a fluorescent dye is being used, the optical slope is essentially independent of the illuminating light intensity over a large range thereof.

The magnitude of these will also depend on the type of illumination: where an illumination yielding strong contrasts in the image is being used, such as dark field illumination, phase contrast illumination or epifluorescence, the optical slopes for example will be larger than when using illuminations which do not give a strong contrast such as standard transmitted light illumination.

In order to increase the contrast and facilitate the identification of objects in a specimen and distinguish between microorganisms and non-microorganismal objects, it is preferred to use various contrasting agents: dyes which stain living matter in particular, e.g. methylene blue, acridine organe, ethidium bromide, etc.; dyes which are capable of distinguishing between one group of microorganisms to another, e.g. gram stain, as well as dyes and reagents which are capable of identifying specifically one group of microorganisms, such as the monoclonal antibodies, subject of Israel Patent Application No 95140, which are specific for *Enterobacteriaceae.*

It should be noted that the absolute value of the optical slope depends on the type of illumination used: where the illumination is of a kind in which particulate objects are viewed as brighter than their surroundings, such as for example, epifluorescent illumination, dark field illumination, etc., the optical slope will have a positive value (the gradient being measured from the inside out) while where the illumination is of a kind in which particulate objects seem darker than their surrounding, e.g. standard field illumination, the optical slope will have a negative value. In addition, the absolute value of the optical slope as will be readily appreciated by a man skilled in the art, depends also on the type of lens used, on the type of microscope, on whether filters are used and the kind of filters, etc.

A person versed in the art will have no difficulties with a limited, straightforward experimentation to calibrate a system having a specific setup in order to determine the range of values of the optical slope which characterize a microorganism. This may be performed for example by determining, under the same conditions, the optical slope of objects in standard suspensions which contain either various non-microorganismal objects and other suspensions which contain microorganisms.

The accuracy of the assay in accordance with the invention may be increased if various other morphological parameters for each object are also determined. Such morphological parameters include the object's length, width and area as well as the overall shape.

The term **"morphological parameters"** to be used at times hereinbelow denotes parameters of an object which are measured in accordance with the present invention including the optical slope, moment, length, width, and the overall shape and any other measured parameter.

By determining various morphological parameters of each object it may be characterized as either a microorganism or a non-microorganismal object on the basis of its position in a multi-dimensional parameter field, the parameters being optical slope and one or more selected from the group consisting of the moment, length, width, area, overall shape as well as any other morphological parameter. In addition to improving the accuracy in determining whether an object is a microorganism or not, the position of an object in such a multidimensional parameter field may enable further characterisation of an object as to its identity, e.g. bacteria, yeast, type of bacteria or yeast etc.

By identifying all microorganisms in one or more viewed images of one or more specimens the concentration of microorganismal cells in the sample may be determined.

The present invention also provides a method for determining the sensitivity of microorganisms in a biological sample to antibiotic drugs. For determining the sensitivity of microorganisms in a sample to such drugs, a specimen of the sample is incubated for a short period of time, e.g. 0.5-2 hour in the presence of the drug and the concentration of the microorganisms in the sample after such an incubation is compared to that of a control specimen from the same sample incubated under the same conditions but without the antibiotic drug.

The present invention thus provides a method for determining the sensitivity of microorganisms to an antibiotic drug which comprises preparing a test mixture comprising the microorganisms and the antibiotic drug and a control mixture comprising the microorganisms, but without the antibiotic drug, incubating both mixture under the same conditions for the same amount of time and determining the concentration of the microorganismal cells after the incubation in both mixtures by the computerised image analysis method of the invention.

Additionally, it was surprisingly found in accordance with the present invention that microorganisms sensitive to an antibiotic drug undergo detectable morphological changes when exposed to this drug. Morphological changes of microorganisms as a result of exposure to antibiotic drugs have been reported (27) but to date no correlation was found to exist between the morphological change and the antibiotic sensitivty (28). It is only with the present invention where for the first time microorganisms were accurately identified and various morphological parameters have been measured on the single cell level that it was found that certain morphological changes of microorganisms which occur as a result exposure to antibiotic drugs are an indication to the sensitivity of microorganisms to such drugs.

The present invention thus provides a method for determining the sensitivity of microorganisms to an antibiotic drug which comprises preparing a test mixture comprising the microorganisms and the antibiotic drug and a control mixture comprising the microorganisms, but without the antibiotic drug, incubating both mixture under the same conditions for the same amount of time, measuring the microorganisms morphological parameters by the computerised image analysis method of the invention and determining the sensitivity on the basis of a change in these parameters between the two mixtures.

For determining the sensitivity of microorganisms to antibiotic drugs, preferably both morphological changes as well as changes in cell concentration after exposure to the drugs are determined and the degree of sensitivity of the microorganism in question to the said drug is determined on the basis of both results.

Also provided by the present invention is an image analysis system for detecting microorganisms in a sample comprising a microscope, means for recording the image viewed through the microscope, means for digitising the recorded image and image processing means characterised in that said image processing means are adapted to classify an object as being either a microorganism or a non-microorganismal object by means of determining the optical slope of the object, the optical slope being the change in brightness across the edge of the object, an optical slope within a certain predetermined range signifying that the object is a microorganism.

Preferably said image processing means are adapted also for analysing various other morphological parameters of the objects detected in the image viewed through the microscope.

The method and system of the present invention thus enable the determination of a microorganismal cell concentration as well as the characterization of microorganisms in a sample. The method and system of the present invention enable also to assess the sensitivity of microorganisms to antibiotic drugs by measuring both cell concentration as well as various morphological parameters with and without exposure to the antibiotic drug.

DETAILED DESCRIPTION OF THE DRAWINGS

The annexed drawings serve as an illustration of the present invention. In the drawings:

Fig 1 gives a general outline of operational steps of the initial characterisation of objects in a sample in accordance with the present invention.

**Figs. 2-4** are more detailed outlines of each of the operational steps identified as blocs I-III in Fig. 1, respectively;

**Fig. 5** is a graphical representation of results of cell concentrations of *E. coli* suspended in buffer, obtained by the method of the present invention (Ordinate) versus actual cell counts (Abcissca). Open squares represent individual results and open triangles average of two or three results.

**Fig. 6** is a graphical representation of cell concentration in 144 milk samples determined by a standard method (Abcissca) versus cell concentrations obtained by the method in accordance with the invention (Ordinate).

**Fig. 7** is a graphical representation of a comparison made between bacterial concentration in urine determined by the standard method (Ordinate) versus results obtained by the method in accordance with the present invention (Abcissca).

**DETAILED DESCRIPTION OF THE INVENTION**

In the following the invention will be illustrated with reference to a non-limiting specific embodiment shown by way of block diagram in the accompanying drawings. A person versed in the art will no doubt appreciate that various modifications of this embodiment as well as other embodiments may be envisaged all being within the scope of the invention as defined above.

For performing the method in accordance with the present invention, a biological sample is first placed under the objective of a microscope and illuminated. Quite generally, any type of illumination may be used but preferred in accordance with the present invention is use such an illumination by which small particulate transparent objects such as bacteria seem brighter than their surroundings, e.g. dark field illumination, phase contract illumination and epifluorescence, the latter being particularly preferred. (Dyes used for epifluorescence may for example, be fluorescein, isothiocyanate, acridine orange, ethidium bromide, propidium iodide, etc). It is furthermore preferred in accordance with the present invention to add various dyes which increase the contrast in the image and thus facilitate the detection of small objects in the sample. Particularly preferred are dyes which are capable of staining living matter, thus facilitating the distinction between cells and other particulate objects, and especially dyes which are capable of staining specifically microorganisms. Most preferred for use in the method of the present invention are dyes which are capable of staining a specific group of microorganisms, such as the *Enterobacteriaceae* antibodies disclosed in the copending Israel Application No. 94140.

It is preferred to flatten and level a sample to be tested so as to be capable of focussing on all objects in a field of view at the same time.

The illuminated and optionally stained sample, is recorded by appropriate image recording means which may, for example, be a video camera an array of photovoltaic cells, a video camera being particularly preferred for the simplicity of its use (including the relative simplicity of the electronic circuit required for recording the image) and its relatively low price. The image is recorded by said image recording means as a multitude of picture elements (pixels), and the brightness in each of said pixels is measured and digitised.

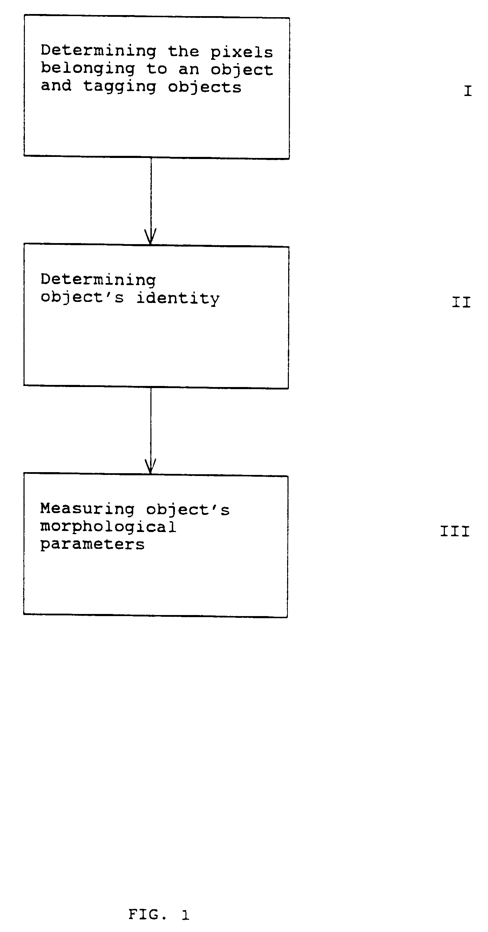

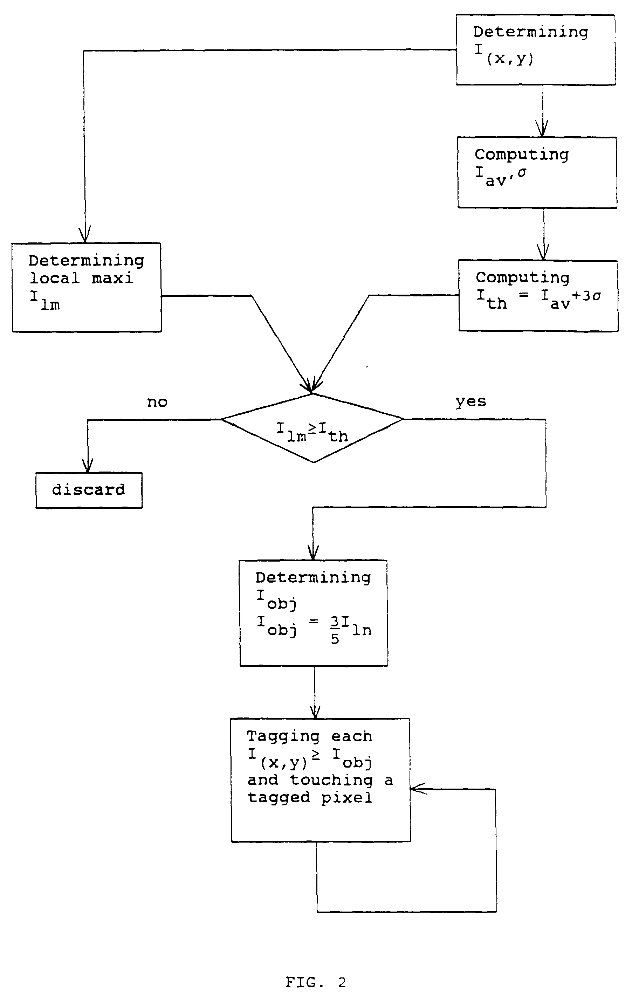

The so-measured and digitised images are then processed as shown generally by way of a very simplified block diagram in Fig. 1. At the first stage I the pixels which form part of object are identified and objects are tagged. Thereafter, at a next stage II, the objects identity, namely whether it is a microorganism or a non-microorganismal object, is determined in accordance with the present invention and then at a final stage III, various morphological parameters of the objects which were identified as microorganisms are determined. Each of the method stages I to III in Fig. 1 are described in somewhat more details hereinbelow with reference to Figs. 2-4, respectively.

Reference is made now to Fig. 2 which describes the manner in which objects are identified and tagged. At the first stage the brightness level in each one of the pixels, $I_{(x,y)}$ [$I_{(x,y)}$ - the brightness level of a pixel having certain x,g coordinates] is measured and therefrom the average brightness of the entire field of view, $I_{av}$, and the standard deviation of the brightness, $\sigma$, are determined. $I_{av}$ and $\sigma$ are used in the next stage to compute the threshold brightness level, $I_{th}$, which is a brightness level used as a reference level in the following stages. $I_{th}$ may be computed as a brightness level higher by a certain amount of standard deviations ($\sigma$), e.g. three in the embodiment shown in Fig. 2, from $I_{av}$.

Alternatively, in a precalibrated system the threshold brightness level, $I_{th}$, may also be a predetermined constant.

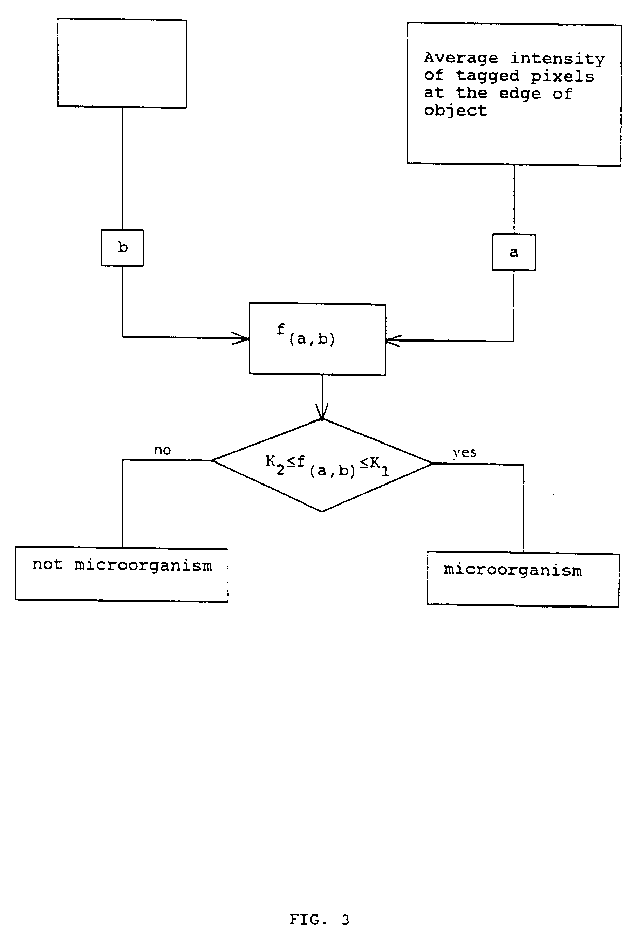

Local maxima, which are pixels the brightness of which is stronger than their eight neighbours, are then identified. The local maxima, $I_{lm}$, are compared to $I_{th}$, those being smaller than $I_{th}$ are discarded while those being

larger than $I_{th}$ are tagged. A brightness level above which an adjacent pixel is considered as belonging to the same object as a tagged pixel, $I_{obj}$, is determined, $I_{obj}$ being a certain fraction of $I_{lm}$, 3/5 in the particular embodiment shown in Figure 3. Then, each pixel which is touching a tagged pixel, and which has a brightness level that is larger or equal to $I_{obj}$ is being tagged and eventually this brings about tagging of all the relevant pixels touching either the local maximum pixel or other tagged pixels. All tagged pixels touching one another are then considered as one object.

By repeating this operation with respect to all the local maxima in the viewed image, all objects are being tagged.

Local maxima which are close to one another, e.g. separated from one another by one or two pixels and the tagged pixels associated therewith are interpreted as belonging to a corresponding number of objects.

The objects identified as above consist of microorganisms as well as non-microorganismal objects such as dust particles, aggregates of macromolecules, cell debris and the like. The present invention is characterised by the manner in which the non-microorganismal objects and microorganisms are distinguished from one another. An example of the manner in which this is being carried out is described in Figure 3.

As mentioned above, it was found in accordance with the present invention that an object may be characterised as a microorganism on the basis of its optical slope, as defined above. If the optical slope falls within a certain predetermined range, which depends to a large extent on the type of illumination and the light intensity, the object is classified as a microorganism, and where the optical slope is either larger or smaller than this range, the object is classified as a non-microorganismal object.

The optical slope may be determined by a number of ways and the specific embodiment shown in Figure 3, is an example only.

For determining the optical slope in accordance with this embodiment, at first stage the average brightness of pixels at the edge of an object, (namely tagged pixels of an object which touch untagged pixels and also the average brightness of all untagged pixels (which are thus not part of the object), but which touch the aforementioned tagged pixels are determined and corresponding values (a) and (b) are obtained. The optical slope is then determined as a function of both (a) and (b), e.g. by computing the difference between (a) and (b) or the ratio of (a) to (b) and the like. The value of this function, $f_{(a,b)}$, is compared to certain predetermined values K1 and K2 and if the object falls within these values it is considered as a microorganism, while where $f_{(a,b)}$ is above or below this range, the object is considered as a non-microorganismal object.

The values K1 and K2 may be determined experimentally by testing known samples containing either various kinds of microorganisms or various kinds of non-microorganismai objects.

For example, using the parameters of the experiment which are described below it was found that where f(a, b) was calculated by the difference between (a) and (b) $[f_{(a,b)} = a-b]$, the values of $k_1$ and $k_2$ where 10 and 1.6 (namely where $f_{(a,b)}$ was between 1.6 and 10 the object was a microorganism) and where $f_{(a,b)}$ was calculated by the ratio of (a) to (b) $[f_{(a,b)} = a/b]$, the values of $k_1$ and $k_2$ where 100 and 25, respectively.

In addition to the optical slope, also other parameters of an object may be determined. Such parameters are for example, the moment, length, width area and overall shape. Such parameters may serve for a better characterisation of an object as a microorganism or a non-microorganismal object on the basis of its position in a multidimensional parameter field. Such position of an object enables also a further characterisation of a microorganism as to its identity.

Determination of the object's length, width and area may be performed as known per se and this will thus not be elaborated herein.

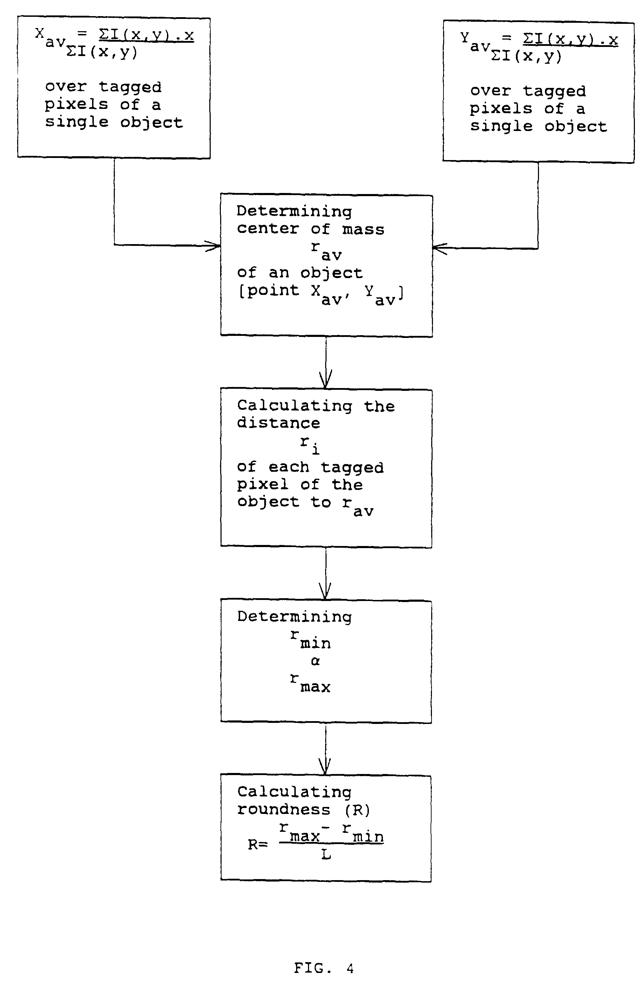

Reference is now made to Fig. 4 which shows, by way of example, one embodiment for the determination of an cbject's overall shape, which is referred to herein as the "roundness". The roundness is a measure of how close the object's shape is to a circle.

At first stage the average X coordinate $X_{av}$, and the average Y coordinate of an object, $Y_{av}$, are determined and the coordinates of the object's centre, $(X_{av}, Y_{av})$, are calculated therefrom. Then the distance, $r_i$, of each tagged pixel of an object to $(X_{av}, Y_{av})$ is calculated, the smallest $r_i$, $r_{min}$, and the largest, $r_{max}$, are determined and the roundness, R, is then calculated from the difference of $r_{max}$ to $r_{min}$ divided by the object's length, L $[R = (r_{max}-r_{min})/L]$ an R of 0 denotes a spherical object while an R close to 0.5 denotes a rod-like object.

A particularly useful parameter in characterising an object as a microorganism and in further characterisation of microorganism's properties is the moment. It was found in accordance with the present invention that the moment of microorganisms differs from that of non-microorganismal objects. The moment may be used for characterizing an object as a microorganism or a non-microorganmismal object, in combination with other parameters mentioned above. In order to characterize an object as a microorganism, the moment of an object has to be within a certain range which is determined experimentally.

The moment (M) may be determined by many ways. By one such way, which is given herein as an example only, the moment is determined in accordance with the following formula:

$$M_{(object)} = \Sigma[r^2_{(x,y)} * I_{(x,y)}]$$

wherein,

$r_{(x,y)}$ is the distance of pixel (x,y) from the object's centre $(x_{av}, y_{av})$;
$I_{(x,y)}$ is the brightness of each pixel (x,y).

As will be appreciated by the artisan, the moment may also be determined by other various other functions of both position and light intensity than the one given herein.

The uniqueness of the method of the present invention is in that in contrast to prior art methods, it is performed by determining properties of individual objects, from which representative values for the entire population (an average and a standard deviation for each one) may be determined. These representative values are characteristic features of a microorganismal population and may at times be used to identify the exact type of microorganisms in a sample. Furthermore, by counting the number of microorganisms identified in an image or in several images of the same sample, the concentration of the microorganisms may be obtained.

The present invention also provides a method for determining the sensitivity of microorganisms to antibiotic drugs. As known, the proliferation of microorganisms is inhibited when exposed to an antibiotic drug to which they are sensitive. Thus, by exposing a specimen of a sample to an antibiotic drug and comparing the cell concentration after such exposure to the concentration of the cells in a control specimen incubated under the same conditions but without the drug, the sensitivity of the microorganisms in the sample to the drug may be assessed. However, in contrast to the prior art method by which a long incubation period was as a rule required, e.g. in the range of 24-36 hours, in accordance with the method of the present invention the required incubation is much shorter, e.g. in a range of 0.5-2 hours. The short incubation period is due to the fact that since individual microorganisms are counted, even a small change in their concentration, which is undetectable by prior art methods, may be identified.

In accordance with the present invention it was surprisingly found that exposure of microorganisms to an antibiotic drug to which they are sensitive, results in morphological changes thereof which are detectable by the method of the invention. Additionally, the occurrence of such changes indicates the sensitivity of a microorganism to the drug which caused the change. In other words, there exists a strong correlation between morphological changes detectable by the method of the invention and the sensitivity of the microorganisms to the drug which causes the change. These morphological changes are particularly apparent when measuring the microorganisms roundness and moment.

Thus, the present invention provides a method for determining the sensitivity of microorganisms to antibiotic drugs comprising incubating the microorganisms for a short period of time, e.g. 0.5-2.0 hours, with an antibiotic drug and determining the degree of change in microorganisms' morphological parameters, in particular the microorganisms roundness and momentum, but also other morphological parameters such as length, width, area and optical slope.

In order to determine the sensitivity of microorganism to antibiotic drugs in accordance with the present invention, a specimen containing the tested microorganism is incubated for a short period of time, e.g. 0.5-2.0 hours, and a control specimen is incubated for the same period of time under the same conditions but without the antibiotic drug. The various properties of the culture, namely the cell concentration and the various morphological parameters are determined both in the tested and in the control specimen and the change in each parameter is determined, e.g. by determining the ratio between the value after exposure to antibiotic drugs (B) to the value of the same parameter without such exposure (A).

Examples of such ratios as measured in accordance with the invention are shown in the following Table I (A:B):

TABLE I

| Parameter | | | | |
|---|---|---|---|---|
| Antibiotic | Length | Area | Moment | Optical Slope |
| Ampicillin | 2.5 | 4 | 12 | 0.5 |
| Spelatine | 2 | 2 | 4 | 0.5 |
| Sulfur | 1.5 | 1.8 | 2.5 | 0.5 |

Each such ratio is then given a score according to its value, e.g. between 0-100. Each of the scores is given a certain weight and thus a weighted average may be calculated. Suitably the score for the change in the microorganism's concentration is given a weight of about 0.4-0.6 and the score for the change in the various morphological parameters is given the rest, e.g. an equal weight to each morphological parameter. This weighted average gives a measure of the microorganisms' sensitivity to the tested antibiotic drug, namely whether the microorganism is sensitive, resistant or intermediate.

Unlike prior art methods of measuring sensitivity of microorganisms to antibiotic drugs, the method in accordance with the present invention may be employed on the original withdrawn biological sample, without any prior incubation stage, even if such a sample contains several kinds of microorganisms. By determining the position of each kind of microorganism in a multidimensional parameter field, the microorganisms may be classified into different populations on the basis of their position in such a multidimensional parameter field and then the change in position as well as in concentration of each population after exposure to an antibiotic drug may be determined. Thereby the antibiotic drug sensitivity of each population within the sample may be determined in a single step.

## DESCRIPTION OF SPECIFIC EMBODIMENTS

The present invention will now be illustrated with reference to some non-limiting specific Examples.

Experimental Procedures

I) Bacterial Preparations

The following bacterial strains were tested:

a) Four standard ATCC strains, *E. coli* 25922; *P. aeruginosa* 27853; *S. faecalis* 29212; *S. aureus* 29213 as recommended by NCCLS, (NCCLS document M7-T2 vol. 8, No, 8, 1988).
b) Strains isolated from urine samples obtained from the bacteriological laboratory of The Sheba Medical Centre and The Zamenhoff Central Laboratory of the General Sick Fund of Israel (Kupat Holim). These strains were isolated and identified in the laboratory using standard procedures as described in the Manual of Clinical Microbiology, 4th edition, 1985. An attempt was made to include the most abundant classes of urinary pathogens. Tested strains included *E. coli, Klebsiella sp. Proteus sp., Pseudomonas aeroginosa* and *Acinetobacter.*

All strains were stored on small glass beads (for reference see Applied Microbiology, Vol. 23, p. 837, 1972) and stored at -20°C. Cultures were initiated by inoculating one bead extracted with sterile tweezers in sterile hood into Muller-Hinton (MH) broth.
Additionally, clinical urine samples and milk samples were also tested.

II) Performing a cell count in accordance with the Standard method :

Preparations were serially diluted and then seeded on agarose plates and after 24 hrs of incubation colonies which developed on the agarose were counted.

III) Assaying Bacterial Samples in accordance with the Present Invention

1 ml from a bacterial preparation was transferred into an Eppendorf tube and 20µl of the nucleic acid binding stain propidium iodide (PI) was added to a final concentration of 50µg/ml and growth of the microorganisms was arrested by heating to 95°C for 5 minutes.
3µl of each of the stained bacterial samples were layered on a microscope slide covered with 18x18mm cover slide and sealed with nail polish (Pavyon clear No. 401).
Slides were then measured using a video-microscopy system in accordance with the present invention. Briefly, a video camera (RCA model 100SU) mounted on an epifluorescent illumination microscope (Olympus Model BH-2) views the sample through a 60x objective and the image is transferred to a microcomputer through an image grabbing printed circuit board. The picture was divided into pixels each one corresponding to a picture area of 0.2 x 0.3µm in size. The light intensity level in each of the pixels was measured and digitised as a value between 0-255. Each slide was scanned by randomly choosing a number of fields (refocusing manually for each field) and the series of images were stored in the computer memory until at least 50 objects with fluorescent values above a predetermined threshold were collected.
The files stored for each sample were copied onto floppy discs and were then analysed off line as follows:
The threshold intensity was determined as 50, (from the overall scale of 0-255), and then tagging the objects, and determining their various morphological parameters was carried out as described above with reference to Figures 1-4. The following parameters were measured for each object, the number against each parameter shows the range of values thereof in which an object was considered a microorganism (all others were considered as non-microorganismal objects).

| Light intensity | 50-250. |
|---|---|
| Object length | 2-15 pixels |
| Object width | 2-4 pixels |
| Optical slope | 1:10-1:2 |
| Roundness | 0-0.5 |
| Moment | 10-100 |

IV) <u>Determination of the antibiotic's sensitivity on the basis of the change in a morphological parameter and change of cell count of the microorganisms.</u>

Bacteria were grown overnight in MH medium and then the preparations were diluted 1000 fold into a fresh MH medium and incubated at 37°C for 2 hours. The culture was then diluted to a final concentration of $1 \times 10^7$/ml and 1ml of this cell suspension was added into a series of tubes containing 1ml of MH medium with a tested antibiotic drug. Three different concentrations of each drug were tested. Three tubes with no drugs served as control: one in which growth was arrested at t=O and two which were incubated concurrently with the drug containing tubes for 120 min. After 120 minutes (and at t=O for one control), 1 ml from each of these tubes was transferred into an Eppendorf tube and then stained with PI and growth was arrested as described above.

In order to assess antibiotic sensitivity each of the above described four morphological parameters was determined both in the tested sample as well as in the control sample and the determination of the degree of sensitivity was carried out as follows:

A) Each morphological parameter was determined for each bacterium identified in the sample.
B) An average for each morphological parameter for all the bacteria in the sample was calculated.
C) The ratio between the average after exposure to antibiotics with the average obtained without exposure to antibiotics was calculated.

If this ratio was larger or smaller (depending on the measured parameters) than a certain first boundary level which was determined specifically for each parameter, it received a score of 100. If this ratio was below or above (again depending on the measured parameters) a certain second boundary (which was also defined for each parameter) it received a score of 0. If the ratio was between these two boundaries, it was given a score between 0-100, linearly dependent on its value between the two boundaries.

The boundaries defined for each of the ratio (listed according to the relevant parameters) is shown in the following Table II:

TABLE II

| PARAMETER | SECOND BOUNDARY (Score of 0) | FIRST BOUNDARY (Score of 100) |
|---|---|---|
| Length | 1.0 | 2.0 |
| Width | 1.0 | 1.5 |
| Optical Slope | 1.0 | 0.75 |
| Moment | 1.0 | 4.0 |
| Roundness | 1.0 | 2.0 |
| Area | 1.0 | 2.0 |

In addition to comparing the morphological parameters between the treated and the control sample as above, also the number of bacteria detected in the 50 arbitrarily chosen fields as described above was compared between the control and the tested sample. The ratio between the numbers in the two cases was determined (treated:control), the minimal boundary was determined at 0.5 and the upper as 0.25. A value between these two boundaries was given a score between 0-100 similarly as above.

Each of the above five scores, namely the four morphological scores and the one corresponding to the change in number was weighted in the following manner: the score of the change in bacterial concentration was given a weighing factor of 0.4 and each of the morphological scores a weighing factor of 0.1. In this manner a weighted average was obtained. The bacteria were considered sensitive if the final weighted average score was 50-100 and were considered resistant if the score was 0-35. The other scores were considered as intermediates.

In order to determine the reproducibility of the method, the standard strains *E. coli* 25922 and *P. aeruginosa*

*27853* were each tested at least 3 times for each of the 5 drugs used in this study. The three tests were carried out on different days each time with a new bacterial preparation in order to avoid bias, two different technicians carried out these experiments which were divided randomly between the two.

V) <u>The standard antimicrobial susceptibility Assay.</u>

The broth dilution antimicrobial susceptibility test was employed as detailed in NCCLS document M7-T2, Vol. 8 No. 8 (1988). Both macro-dilution and micro-dilution assays were carried out in parallel for each tested microorganism. Mueller-Hinton (MH) (Difco) was used throughout the test. The standard *E. coli* ATCC 25922 strain was used as an internal control for each test. Classification of each strain as either resistant (R), sensitive (S) or intermediate (I) was based on comparing the MIC results obtained with the MIC interpretive standards set forth by NCCLS.

**EXAMPLE 1**: <u>Obtaining a cell count by the method of the present invention</u>

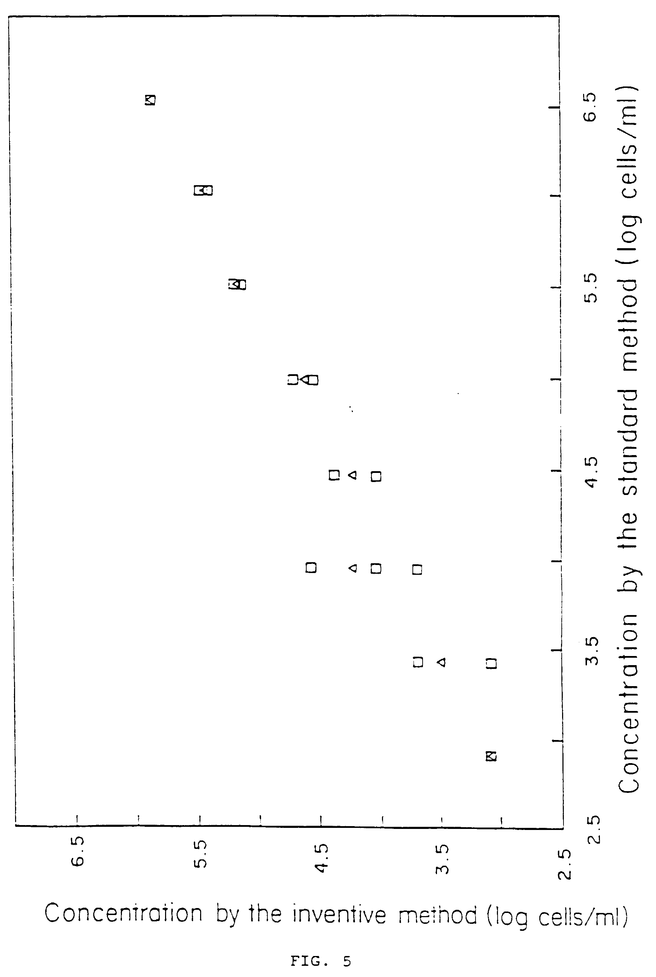

Bacterial cells were suspended in phosphate buffered saline (PBS) in various concentrations of cell/ml. A sample of each suspension was then prepared for microscopy and tested in accordance with the method of the present invention as described in detail above. For some concentrations two or three repetitions were made. The results are shown in Fig. 5 in which the abscissca gives actual cell numbers while the ordinate gives cell counts, as determined in accordance with the method of the invention. (open squares show individual results and open triangles average of the individual results at the same cell concentration).
It may be seen that although the measured cell counts slightly deviate from the actual cell number at higher concentrations, the overall agreement between actual and measured cell counts is remarkable.
It should be noted that the deviation may be easily compensated by a correction factor for higher cell counts.

**EXAMPLE 2**: <u>Determining cell counts in milk</u>

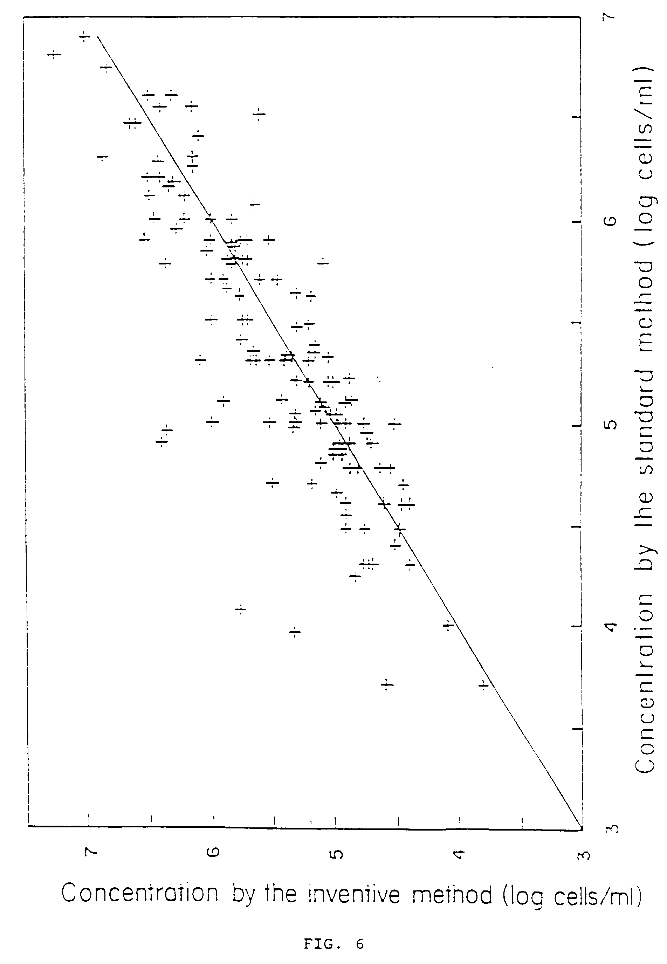

144 fresh milk samples were tested to determine cell counts both by the method of the present invention and by the standard plating method described above. The results obtained are shown in Figure 6 (abscissca-cell count obtained by the standard method; ordinate cell count-obtained by the method of the present invention).
As can be seen both cell counts are in excellent agreement with one another.

**EXAMPLE 3**: <u>Comparison of cell counts in clinical sample obtained by a standard method versus the method in accordance with the resent invention.</u>

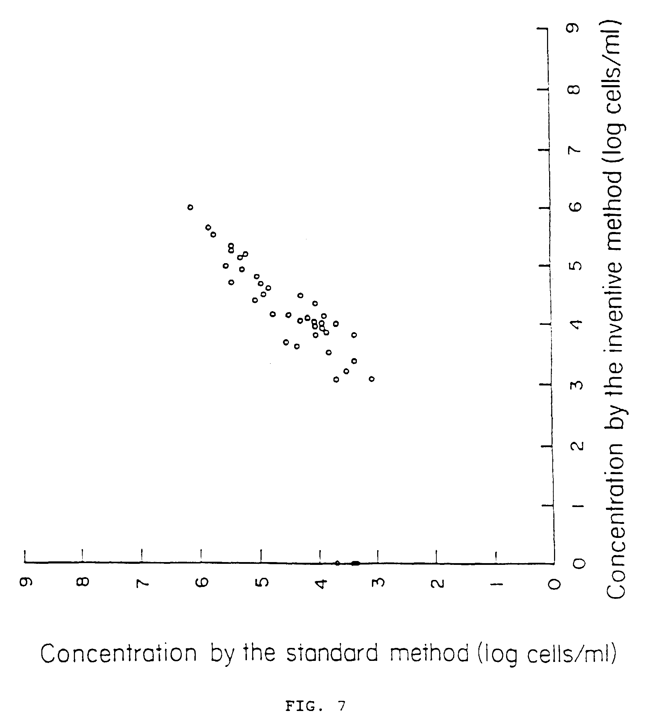

Cell counts in 24 urine samples obtained from various individuals were determined both by standard methods and by the method of the present invention.
A graphical representation of the cell count obtained by the method of the invention versus a cell count obtained by the standard plating method is shown in Fig. 7 (abscissa - cell count in accordance with the invention; ordinate - cell count by the standard method).
Again it may be seen that the results of both methods are in excellent agreement with one another.

**EXAMPLE 4**: <u>Determination of the susceptibility of bacteria to antibiotic drugs</u>

A) <u>Comparison of the method in accordance with the present invention with standard prior art method for determining antibiotic sensitivity.</u>

Various bacterial strains, both standard ATCC strains as well as such isolated by the inventor from clinical samples were tested for sensitivity to 5 common antibiotic drugs, both by the method of the present invention as well as by the standard broth dilution method.
The results obtained with various antibiotic drugs are summarised in the following Tables III-VII (each Table represents an experiment with a different antibiotic drug).

TABLE III

| BACT. | BACTERIA | MICS | SENSITIVITY | | |
|---|---|---|---|---|---|
| Cephalothin | | | | | |
| | | | Std. | Inv. | |
| ATCC | E. coli | 10 | S | S | 39 |
| E165 | E. coli | 10 | S | S | 100 |
| 8227 | E. coli | 10 | S | S | 40 |
| 8492 | E. coli | 10 | S | S | 100 |
| 8494 | E. coli | 3 | S | S | 40 |
| A8562 | E. coli | >64 | R | R | 0 |
| K110 | Klebsiella | >100 | R | R | 2 |
| KATCC | Klebsiella | 10 | S | S | 98 |
| M197 | Proteus | 3 | S | S | 71 |
| V188 | Proteus | 10 | S | S | 47 |
| 3766 | Proteus | >100 | R | S | 100 |
| 3728 | Proteus | >100 | R | R | 0 |
| P138 | Pseudomonas | >100 | R | R | 10 |
| PATCC | Pseudomonas | >32 | R | R | 5 |

BACT - Inventor's Bacteria Designation

MICS - Minimal inhibitory concentration (in µg/ml) obtained by the standard broth dilution method.

Std. - Standard method

Inv. - Method of the invention R - Resistant; S - Sensitive

Score - Score obtained by the method of the invention.

TABLE IV

| BACT. | BACTERIA | MICS | SENSITIVITY | | SCORE |
|---|---|---|---|---|---|
| (Ampicillin) | | | | | |
| | | | Std. | Inv. | |
| A117 | Enterob | >32 | R | R | 7 |
| ATCC | E. coli | 4 | S | S | 90 |
| ATCC | E. coli | 4 | S | S | 94 |
| ATCC | E. coli | 4 | S | S | 75 |
| E119 | E. coli | >32 | R | R | 0 |
| E128 | E. coli | 4 | S | S | 85 |
| E148 | E. coli | >32 | R | R | 0 |
| E163 | E. coli | 4 | S | S | 98 |
| E165 | E. coli | 4 | S | S | 100 |
| K110 | Klebsiella | 10 | S | S | 100 |
| KATCC | Klebsiella | >100 | R | R | 14 |
| P3480 | Proteus | >100 | R | R | 3 |
| P3499 | Proteus | >100 | R | R | 1 |
| P3719 | Proteus | 1 | S | S | 100 |

TABLE IV   (continued)

| (Ampicillin) | | | | | |
|---|---|---|---|---|---|
| BACT. | BACTERIA | MICS | SENSITIVITY | | SCORE |
| | | | Std. | Inv. | |
| P3728 | Proteus | 1 | S | S | 100 |
| See Footnote Table III | | | | | |

TABLE V

| (Nalidixic acid) | | | | | |
|---|---|---|---|---|---|
| BACT. | BACTERIA | MICS | SENSITIVITY | | SCORE |
| | | | Std. | Inv. | |
| ATCC | E. coli | 2 | S | S | 95 |
| E1134 | E. coli | >64 | R | R | 13 |
| E128 | E. coli | 4 | S | S | 100 |
| E165 | E. coli | 8 | S | S | 100 |
| E184 | E. coli | 8 | S | S | 86 |
| E185 | E. coli | 8 | S | S | 100 |
| E904 | E. coli | >64 | R | R | 2 |
| E956 | E. coli | >64 | R | R | 0 |
| KATCC | Klebsiella | 2 | S | S | 99 |
| K110 | Klebsiella | 8 | S | S | 49 |
| 3766 | Proteus | 8 | S | S | 60 |
| 3780 | Proteus | >64 | R | R | 12 |
| See Footnote Table III | | | | | |

TABLE VI

| (Trimethoprim-Sulfamethoxazole) | | | | | |
|---|---|---|---|---|---|
| BACT. | BACTERIA | MICS | SENSITIVITY | | SCORE |
| | | | Std. | Inv. | |
| E165 | E. coli | >32 | R | R | 1 |
| E2222 | E. coli | 0.3 | S | S | 100 |
| E3319 | E. coli | 3 | S | S | 55 |
| E3347 | E. coli | >100 | R | R | 3 |
| E3347 | E. coli | >32 | R | R | 3 |
| E163 | E. coli | 1 | S | S | 86 |
| EATCC | E. coli | 0.5 | S | S | 84 |
| EATCC | E. coli | 0.5 | S | S | 99 |
| K110 | Klebsiella | 10 | S | S | 68 |
| KATCC | Klebsiella | 3 | S | S | 100 |
| M197 | Proteus | 3 | S | S | 97 |
| V188 | Proteus | 1 | S | S | 51 |
| 3766 | Proteus | >100 | R | R | 4 |
| 3480 | Proteus | >100 | R | R | 26 |

TABLE VI   (continued)

| (Trimethoprim-Sulfamethoxazole) | | | | | |
|---|---|---|---|---|---|
| BACT. | BACTERIA | MICS | SENSITIVITY | | SCORE |
| | | | Std. | Inv. | |
| 3499 | Proteus | 3 | S | S | 100 |
| P138 | Pseudomonas | >32 | R | R | 0 |
| PATCC | Pseudomonas | >32 | R | R | 2 |
| See Footnote Table III | | | | | |

TABLE VII

| (Gentamicin) | | | | | |
|---|---|---|---|---|---|
| BACT. | BACTERIA | MICS | SENSITIVITY | | SCORE |
| | | | Std. | Inv. | |
| A8562 | Abacter | >100 | R | R | 0 |
| ATCC | E. coli | 1 | S | S | 73 |
| E163 | E. coli | 0.5 | S | S | 67 |
| E185 | E. coli | 1 | S | S | 87 |
| K110 | Klebsiella | 1-4 | S | R | 17 |
| KATCC | Klebsiella | 0.5 | S | S | 80 |
| V188 | Proteus | 1 | S | S | 67 |
| P3601 | Pseudomonas | >100 | R | R | 2 |
| PATCC | Pseudomonas | 1 | S | S | 60 |
| See Footnote Table III | | | | | |

B) Reproducibility Experiments

In order to evaluate the degree of reproducibility of the method in accordance with the present invention two standard NCCLS recommended strains *E. coli* ATCC 25922 and *Pseudomonas aeruginos* a 27853 were tested for each of the five drugs tested in A above. Each test with each strain was repeated three times, independent repeat performed on different days by different laboratory technicians. The results are shown in the following Tables VIII-XI.

TABLE VIII

| (Trimethoprim-Sulfamethoxazole) | | | | |
|---|---|---|---|---|
| BACTERIA | MICS | SENSITIVITY | | SCORE |
| | | Std. | Inv. | |
| E. coli | 0.5 | S | S | 99 |
| E. coli | 0.5 | S | S | 81 |
| E. coli | 0.5 | S | S | 61 |
| Pseudomonas | >32 | R | R | 2 |
| Pseudomonas | >32 | R | R | 1 |
| Pseudomonas | >32 | R | R | 0 |
| See Footnote Table III | | | | |

TABLE IX

| (Ampicillin) | | | | |
|---|---|---|---|---|
| BACTERIA | MICS | SENSITIVITY | | SCORE |
| | | Std. | Inv. | |
| E. coli | 4 | S | S | 90 |
| E. coli | 4 | S | S | 94 |
| E. coli | 4 | S | S | 75 |
| E. coli | 4 | S | S | 90 |
| E. coli | 4 | S | S | 98 |
| E. coli | 4 | S | S | 100 |
| See Footnote Table III | | | | |

TABLE X

| (Gentamicin) | | | | |
|---|---|---|---|---|
| BACTERIA | MICS | SENSITIVITY | | SCORE |
| | | Std. | Inv. | |
| E. coli | 1 | S | S | 73 |
| E. coli | 1 | S | S | 100 |
| E. coli | 1 | S | S | 100 |
| Pseudomonas | 1 | S | S | 60 |
| Pseudomonas | 1 | S | S | 60 |
| Pseudomonas | 1 | S | S | 60 |
| See Footnote Table III | | | | |

TABLE XI

| (Nalidixic Acid) | | | | |
|---|---|---|---|---|
| BACTERIA | MICS | SENSITIVITY | | SCORE |
| | | Std. | Inv. | |
| E. coli | 2 | S | S | 95 |
| E. coli | 2 | S | S | 100 |
| E. coli | 2 | S | S | 81 |
| Pseudomonas | >128 | R | R | 2 |
| Pseudomonas | >128 | R | R | 2 |
| Pseudomonas | >128 | R | R | 4 |
| See Footnote Table III | | | | |

As can be seen in this Example, the method in accordance with the present invention classified the standard strains correctly as susceptible or resistant in almost all cases. In 96% (49/51) of the cases in which urinary isolates were examined, the method of the present invention yielded results identical with those obtained by the standard method (microdilution and macrodilution which were performed in parallel on each sample) in terms of susceptibility or resistance. 4% (2/51 of the cases) were determined incorrectly by the method of the present invention as compared to the standard method: one case was falsely determined as sensitive and one case falsely determined as resistant.

As described above, the results were obtained after 2 hours of incubation with the antibiotic drug. However, the incubation period may be considerably reduced.

**LIST OF REFERENCES**

1. Isenberg et al, 1985. In: "Manual of Clinical Microbiology", Edwin H. Lennette, Albert Balows, William J. Hausler, J.R., M. Jean Shadomy, (eds.) 4th Ed. American Society for Microbiology, Washington, D.C. p: 73-98.

2. Washington et al., Ibid, p. 967-987.

3. Memorandum from a WHO Meeting. 1983. Bulletin of the World Health Organization, 61 (3): 423-433.

4. Pezzlo et al., 1982. J. Clin. Microbiol. 15: 468-474.

5. Bates 1982. Lab. Manag. 20:7-13.

6. Donta et al., 1981. N. England J. Med. 304: 939-943.

7. Kass 1978. J. Infect. Dis. 138: 546-557.

8. D'Amato et al., 1985. In: Manual of Clinical Microbiology. Leunette C.H., Balows A., Hauslower W.J. Jr., Shadomy H.J. (eds.). American Society for Microbiology, Washington, D.C., pp. 52-65.

9. Thornsberg et al., 1985. Ibid., pp. 1015-1018.

10. Baird-Parker 1989. In: Rapid Methods and Automation in Microbiology and Immunology. Brixia Academic Press, Brescia, Italy, 276-281. Balows A., Tilton R.C., Turano A. (eds.),

11. Isenberg, 1989. Ibid, 320-326.

12. Vincent et al., 1989. Ibid, 325-332.

13. Sanbolle et al., 1989. Ibid, 333-341.

14. Thabaut 1989. Ibid, 342-352.

15. Johnston, 1989. Ibid, 353-359.

16. Kelly et al., 1981. J. Clin. Microbiol. 13:677-680.

17. Hale et al., 1981. J. Clin. Microbiol. 13:147-150.

18. Aldrige et al., 1977. J. Clin. Microbiol. 6: 406-413.

19. Pfaller, 1985. J. Clin. Microbiol. 21: 783-787.

20. Bixler-Forell et al. 1985. J. Clin. Microbiol. 22:62-67.

21. Cady et al., 1978. J. Clin. Microbiol. 7:273-278.

22. Morgan et al., 1983. J. Clin. Microbiol. 18: 384-388.

23. Wu et al., 1985. J. Clin. Microbiol. 21:796-799.

24. Wallis et al., 1981. J. Clin. Microbiol. 14:342-346.

25. Perry et al., 1982., J. Clin. Microbiol. 15:852-854.

26. Marr et al. 1975. Am. J. Dis. Child. 129:940-943.

27. Atkinson et al., 1984. In: Antimicrobial Therapy, A.M. Ristuccia and B.A. Cunha (eds.) Rowen Press, New York, pp. 23-36.

28. Greenwood, 1985. In: Rapid Methods and Automation in Microbiology and Immunology, K.O. Habermehe (ed.), Springer Verlag, Berlin, pp. 479-509.

## Claims

1. A computerized image analysis method for detecting microorganisms in a sample comprising placing a specimen from the sample in a microscope, recording the image viewed through the microscope by appropriate image recording means, analyzing the image to identify particulate objects therein and classifying these objects as microorganisms or non-microorganismal objects, which classifying comprises determining the optical slope being the change in brightness across the edge of the object.

2. A method according to Claim 1, wherein the classification comprises establishing whether said optical slope falls within a certain range, an object which is classified as a microorganism having said optical slope within said range.

3. A method according to Claim 2, wherein said classifying further comprises determining the moment, being a product of the light intensity of each point in the object and its distance from the object's center.

4. A method according to Claim 3, wherein the classification comprises establishing whether said moment falls within a certain range, an object which is classified as a microorganism having said brightness variance within said range.

5. A method according to any one of Claims 1 to 4, wherein the classification of an object as a microorganism comprises also the determination of the object's length, width, area and its overall shape.

6. A method according to Claim 5, wherein the characterization of the object as a microorganism is based on its position in a multidimensional parameter field, the parameters comprising said optical slope, said moment, length, width and overall shape.

7. A method according to Claim 6, wherein the object's position in said multidimensional parameter field is used to further characterize the type of the microorganism.

8. A method according to any one of Claims 1 to 7, comprising determining the concentration of microorganisms in a sample.

9. A method for determining the sensitivity of microorganisms to an antibiotic drug which comprises:

   (a) preparing a test mixture comprising the microorganisms and the antibiotic drugs and a control mixture comprising the microorganisms, but without the antibiotic drug, incubating both mixtures under the same conditions for the same amount of time;
   (b) detecting microorganisms in said test mixture and in said control mixture by the method according to any one of Claims 1 to 7 and measuring at least one other morphological parameter of the detected microorganisms;
   (c) comparing microorganismal count in said test mixture and in said control mixture and determining sensitivity of the microorganisms to the antibiotic drug based on the difference in this parameter in said test mixture as compared to said control mixture.

10. A method for determining the sensitivity of microorganisms to an antibiotic drug which comprises:

   (a) preparing a test mixture comprising the microorganisms and the antibiotic drugs and a control mixture comprising the microorganisms but without the antibiotic drug, incubating both mixtures under the same conditions for the same amount of time;
   (b) detecting microorganisms in said test mixture and in said control mixture by the method according to any one of Claims 1 to 7 and determining the count of detected microorganisms in each of the mixtures;
   (c) comparing microorganismal count in said test mixture and in said control mixture and determining sensitivity of the microorganisms to the antibiotic drug based on the difference in this parameter in said test mixture as compared to said control mixture.

11. A method according to Claim 10 wherein the determination of sensitivity to antibiotics also comprises determining at least one other morphological parameter of the microorganism and comparing the change in said morphological parameter as between the two mixtures.

12. A method according to Claim 9 or 11, wherein said at least one other morphological parameter is selected from the group consisting of the object's length, width, area, overall shape and moment.

13. An image analysis system for detecting microorganisms in a sample, comprising a microscope, means for recording an image viewed through the microscope, means for digitizing the recorded image, and image processing means; the system being characterized in that said image processing means are adapted to classify an object as being either a microorganism or a non-microorganismal object by means of determining the optical slope of the object, the optical slope being the change in brightness across the edge of the object, an optical slope within a certain predetermined range signifying that the object is a microorganism.

14. A system according to Claim 13, wherein said image processing means are further adapted to determine the moment of the object, the moment being a product of the light intensity of each point in the object and the distance from the object's center, a moment within a predetermined range signifying that the object is a microorganism.

15. A system according to Claim 13 or 14, wherein said image processing means are further adapted to classify an object as a microorganism on the basis of an object's length, width, area and its overall shape.

16. A system according to any one of Claims 13 to 15, wherein said image processing means are adapted to determine the position of an object in a multi-dimensional parameter field, the parameters comprising said optical slope, said moment, length, width and overall shape.


**Patentansprüche**

1. Verfahren zur computerisierten Bildanalyse zum Nachweis von Mikroorganismen in einer Probe, wobei man eine Probe aus der Probe in ein Mikroskop einbringt, das durch das Mikroskop betrachtete Bild durch eine geeignete Bildaufzeichnungsvorrichtung aufzeichnet, das Bild analysiert, um teilchenförmige Objekte darin zu identifizieren und diese Objekte als Mikroorganismen oder nicht zu Mikroorganismen gehörige Objekte zu klassifizieren, wobei man bei der Klassifizierung das optische Gefälle bestimmt, das die Veränderung der Helligkeit über die Kante des Objekts ist.

2. Verfahren nach Anspruch 1, wobei man bei der Klassifizierung feststellt, ob das optische Gefälle in einen bestimmten Bereich fällt, wobei ein Objekt als Mikroorganismus klassifiziert wird, das das optische Gefälle in diesem Bereich besitzt.

3. Verfahren nach Anspruch 2, wobei man bei der Klassifizierung weiter das Moment bestimmt, das ein Produkt aus der Lichtintensität jedes Punkts in dem Objekt und seine Entfernung vom Mittelpunkt des Objekts ist.

4. Verfahren nach Anspruch 3, wobei man bei der Klassifizierung feststellt, ob das Moment in einen bestimmten Bereich fällt, wobei ein Objekt, das als Mikroorganismus klassifiziert ist, die Helligkeitsvarianz innerhalb dieses Bereichs besitzt.

5. Verfahren nach einem der Ansprüche 1 bis 4, wobei man bei der Klassifizierung eines Objekts als Mikroorganismus auch die Länge, Breite, Fläche und dessen Gesamtform bestimmt.

6. Verfahren nach Anspruch 5, wobei die Charakterisierung des Objekts als Mikroorganismus auf seiner Position in einem multidimensionalen Parameterfeld beruht, wobei die Parameter das optische Gefälle, das Moment, die Länge, die Breite und die Gesamtform umfassen.

7. Verfahren nach Anspruch 6, wobei die Position des Objekts in dem multidimensionalen Parameterfeld zur weiteren Charakterisierung des Typs des Mikroorganismus verwendet wird.

**8.** Verfahren nach einem der Ansprüche 1 bis 7, wobei man die Konzentration der Mikroorganismen in einer Probe bestimmt.

**9.** Verfahren zur Bestimmung der Empfindlichkeit von Mikroorganismen gegenüber einem Antibiotikum, wobei man:

(a) ein Testgemisch aus den Mikroorganismen und den Antibiotika und ein Kontrollgemisch aus den Mikroorganismen, aber ohne Antibiotikum, herstellt, beide Gemische unter den gleichen Bedingungen für die gleiche Zeitspanne inkubiert;

(b) die Mikroorganismen in dem Testgemisch und in dem Kontrollgemisch nach dem Verfahren nach einem der Ansprüche 1 bis 7 nachweist und mindestens einen weiteren morphologischen Parameter der nachgewiesenen Mikroorganismen mißt;

(c) die Mikroorganismenzahl in dem Testgemisch und in dem Kontrollgemisch vergleicht und die Empfindlichkeit der Mikroorganismen gegenüber dem Antibiotikum auf der Grundlage des Unterschieds in diesem Parameter in dem Testgemisch verglichen mit dem Kontrollgemisch bestimmt.

**10.** Verfahren zur Bestimmung der Empfindlichkeit von Mikroorganismen gegenüber einem Antibiotikum, wobei man:

(a) ein Testgemisch aus Mikroorganismen und den Antibiotika und ein Kontrollgemisch aus den Mikroorganismen, aber ohne Antibiotikum, herstellt, beide Gemische unter den gleichen Bedingungen für die gleiche Zeitspanne inkubiert;

(b) die Mikroorganismen in dem Testgemisch und in dem Kontrollgemisch nach dem Verfahren nach einem der Ansprüche 1 bis 7 nachweist und die Zahl der nachgewiesenen Mikroorganismen in jedem der Gemische bestimmt;

(c) die Mikroorganismenzahl in dem Testgemisch und in dem Kontrollgemisch vergleicht und die Empfindlichkeit der Mikroorganismen gegenüber dem Antibiotikum auf der Grundlage des Unterschieds in diesem Parameter in dem Testgemisch verglichen mit dem Kontrollgemisch bestimmt.

**11.** Verfahren nach Anspruch 10, wobei man bei der Bestimmung der Empfindlichkeit gegenüber Antibiotika noch mindestens einen weiteren morphologischen Parameter der Mikroorganismen bestimmt und die Veränderung bezüglich dieses morphologischen Parameters zwischen den zwei Gemischen vergleicht.

**12.** Verfahren nach Anspruch 9 oder 11, wobei der mindestens eine weitere morphologische Parameter aus der Gruppe, bestehend aus der Länge, der Breite, der Fläche, der Gesamtform und dem Moment des Objekts, ausgewählt wird.

**13.** Bildanalysesystem zum Nachweis von Mikroorganismen in einer Probe, umfassend ein Mikroskop, Mittel zur Aufzeichnung eines durch das Mikroskop betrachteten Bilds, Mittel zur Digitalisierung des aufgezeichneten Bilds und Bildverarbeitungsmittel, wobei das System dadurch **gekennzeichnet** ist, das die Bildverarbeitungsmittel der Klassifizierung eines Objekts als entweder ein Mikroorganismus oder ein nicht zu einem Mikroorganismus gehöriges Objekt durch Bestimmung des optischen Gefälles des Objekts angepaßt ist, wobei das optische Gefälle die Helligkeitsveränderung über die Kante des Objekts ist, wobei ein optisches Gefälle innerhalb eines bestimmten vorbestimmten Bereichs anzeigt, daß das Objekt ein Mikroorganismus ist.

**14.** System nach Anspruch 13, wobei das Bildverarbeitungsmittel weiter der Bestimmung des Moments des Objekts angepaßt ist, wobei das Moment ein Produkt aus der Lichtintensität jedes Punkts in dem Objekt und der Entfernung vom Mittelpunkt des Objekts ist, wobei ein Moment innerhalb eines vorbestimmten Bereichs anzeigt, daß das Objekt ein Mikroorganismus ist.

**15.** System nach Anspruch 13 oder 14, wobei die Bildverarbeitungsmittel weiter der Klassifizierung eines Objekts als Mikroorganismus auf der Basis der Länge, Breite, Fläche und Gesamtform des Objekts angepaßt sind.

**16.** System nach einem der Ansprüche 13 bis 15, wobei die Bildverarbeitungsmittel der Bestimmung der Position eines Objekts in einem multidimensionalen Parameterfeld angepaßt sind, wobei die Parameter die optische Neigung, das Moment, die Länge, die Breite und die Gesamtform umfassen.

**Revendications**

1. Procédé informatisé d'analyse d'image, pour détecter des micro-organismes dans un échantillon, qui comprend la mise en place d'un spécimen provenant de l'échantillon dans un microscope, l'enregistrement de l'image observée à travers le microscope grâce à un moyen approprié d'enregistrement d'image, l'analyse de l'image pour identifier les objets particuliers qui s'y trouvent, et la classification de ces objets en micro-organismes et objets autres que des micro-organismes, cette classification comprenant la détermination de la pente optique, qui est la variation de luminosité à travers le bord de l'objet.

2. Procédé selon la revendication 1, dans lequel la classification consiste à établir si ladite pente optique entre dans un certain intervalle, un objet classé comme étant un micro-organisme ayant une pente optique entrant dans ledit intervalle.

3. Procédé selon la revendication 2, dans lequel ladite classification consiste en outre à déterminer le moment, qui est le produit de l'intensité lumineuse en chaque point de l'objet par sa distance au centre de l'objet.

4. Procédé selon la revendication 3, dans lequel la classification consiste à établir si ledit moment entre dans un certain intervalle, un objet classé comme étant un micro-organisme ayant une variation de luminosité entrant dans cet intervalle.

5. Procédé selon l'une quelconque des revendications 1 à 4, dans lequel la classification d'un objet comme étant un micro-organisme comprend aussi la détermination de la longueur, de la largeur, de l'aire et de la forme globale de l'objet.

6. Procédé selon la revendication 5, dans lequel la caractérisation de l'objet comme étant un micro-organisme se fonde sur sa position dans un champ de paramètres multidimensionnels, les paramètres comprenant ladite pente optique, lesdits moment, longueur, largeur et forme globale.

7. Procédé selon la revendication 6, dans lequel la position de l'objet, dans ledit champ de paramètres multidimensionnels, est utilisée pour caractériser plus avant le type du micro-organisme.

8. Procédé selon l'une quelconque des revendications 1 à 7, qui comprend la détermination de la concentration des micro-organismes dans un échantillon.

9. Procédé pour déterminer la sensibilité de micro-organismes à un médicament antibiotique, qui comprend :

   (a) la préparation d'un mélange d'essai, comprenant les micro-organismes et les médicaments antibiotiques, et un mélange témoin comprenant les micro-organismes mais sans le médicament antibiotique, l'incubation des deux mélanges dans les mêmes conditions et pendant le même laps de temps ;
   (b) la détection de micro-organismes dans ledit mélange d'essai et dans ledit mélange témoin par le procédé selon l'une quelconque des revendications 1 à 7, et la mesure d'au moins un autre paramètre morphologique des micro-organismes détectés ;
   (c) la comparaison du compte de micro-organismes dans ledit mélange d'essai et dans ledit mélange témoin, et la détermination de la sensibilité des micro-organismes aux médicaments antibiotiques sur la base de la différence que présente ce paramètre entre ledit mélange d'essai et ledit mélange témoin.

10. Procédé pour déterminer la sensibilité de micro-organismes à un médicament antibiotique, qui comprend :

   (a) la préparation d'un mélange d'essai, comprenant les micro-organismes et les médicaments antibiotiques, et un mélange témoin comprenant les micro-organismes mais sans le médicament antibiotique, l'incubation des deux mélanges dans les mêmes conditions et pendant le même laps de temps ;
   (b) la détection de micro-organismes dans ledit mélange d'essai et dans ledit mélange témoin par le procédé selon l'une quelconque des revendications 1 à 7, et la détermination du compte des micro-organismes détectés dans chacun des mélanges ;
   (c) la comparaison du compte de micro-organismes dans ledit mélange d'essai et dans ledit mélange témoin, et la détermination de la sensibilité des micro-organismes aux médicaments antibiotiques sur la base de la différence que présente ce paramètre entre ledit mélange d'essai et ledit mélange témoin.

**11.** Procédé selon la revendication 10, dans lequel la détermination de la sensibilité aux antibiotiques comprend aussi la détermination d'au moins un autre paramètre morphologique du micro-organisme, et la comparaison de la variation dudit paramètre morphologique entre les deux mélanges.

**12.** Procédé selon la revendication 9 ou 11, dans lequel au moins ledit autre paramètre morphologique est choisi dans l'ensemble comprenant la longueur, la largeur, l'aire, la forme globale et le moment de l'objet.

**13.** Système d'analyse d'image pour détecter des micro-organismes dans un échantillon, comprenant un microscope, un moyen pour enregistrer une image observée à travers le microscope, un moyen pour numériser l'image enregistrée, et un moyen de traitement d'image ; le système étant caractérisé en ce que ledit moyen de traitement d'image est adapté pour classer un objet comme étant un micro-organisme ou un objet autre qu'un micro-organisme, par l'intermédiaire de la détermination de la pente optique de l'objet, la pente optique étant la variation de luminosité à travers le bord de l'objet, une pente optique entrant dans un certain intervalle prédéterminé signifiant que l'objet est un micro-organisme.

**14.** Système selon la revendication 13, dans lequel ledit moyen de traitement d'image est en outre adapté de façon à déterminer le moment de l'objet, le moment étant le produit de l'intensité lumineuse en chaque point de l'objet par la distance au centre de l'objet, un moment entrant dans un intervalle prédéterminé signifiant que l'objet est un micro-organisme.

**15.** Système selon la revendication 13 ou 14, dans lequel ledit moyen de traitement d'image est en outre adapté de façon à classer un objet comme étant un micro-organisme, sur la base de la longueur, de la largeur, de l'aire et de la forme globale d'un objet.

**16.** Système selon l'une quelconque des revendications 13 à 15, dans lequel ledit moyen de traitement d'image est adapté de façon à déterminer la position d'un objet dans un champ de paramètres multidimensionnels, les paramètres comprenant ladite pente optique, lesdits moment, longueur, largeur et forme globale.

```
┌─────────────────────────────┐
│                             │
│  Determining the pixels     │
│  belonging to an object     │                    I
│  and tagging objects        │
│                             │
│                             │
└─────────────────────────────┘
               │
               ▼
┌─────────────────────────────┐
│                             │
│  Determining                │
│  object's identity          │                    II
│                             │
│                             │
│                             │
└─────────────────────────────┘
               │
               ▼
┌─────────────────────────────┐
│                             │
│  Measuring object's         │
│  morphological              │                    III
│  parameters                 │
│                             │
│                             │
└─────────────────────────────┘
```

FIG. 1

FIG. 2

Average intensity
of tagged pixels
at the edge of
object

b

a

$f_{(a,b)}$

no $\qquad$ $K_2 \leq f_{(a,b)} \leq K_1$ $\qquad$ yes

not microorganism

microorganism

FIG. 3

$$X_{av} = \frac{\Sigma I(x,y).x}{\Sigma I(x,y)}$$

over tagged
pixels of a
single object

$$Y_{av} = \frac{\Sigma I(x,y).x}{\Sigma I(x,y)}$$

over tagged
pixels of a
single object

Determining
center of mass
$$r_{av}$$
of an object
[point $X_{av}$, $Y_{av}$]

Calculating the
distance
$$r_i$$
of each tagged
pixel of the
object to $r_{av}$

Determining
$$r_{min}$$
$$\alpha$$
$$r_{max}$$

Calculating
roundness (R)

$$R = \frac{r_{max} - r_{min}}{L}$$

FIG. 4

FIG. 5

FIG. 6

FIG. 7